# EUROPEAN PATENT SPECIFICATION

(11) **EP 3 155 908 B1**
(45) Date of publication and mention of the grant of the patent: **05.12.2018**
(21) Application number: 15190221.0
(22) Date of filing: 16.10.2015
(51) Int. Cl.: A24F 47/00

(54) **ELECTRONIC SMOKING DEVICE WITH ADAPTABLE ATOMIZING CHAMBER**
ELEKTRONISCHE RAUCHVORRICHTUNG MIT ANPASSBARER SPRÜHKAMMER
DISPOSITIF À FUMER ÉLECTRONIQUE AVEC CHAMBRE D'ATOMISATION ADAPTABLE

(43) Date of publication of application: 19.04.2017
(73) Proprietor: Fontem Holdings 1 B.V., 1083 HN Amsterdam (NL)
(72) Inventor: Borkovec, Vaclav, 22761 Hamburg (DE)
(74) Representative: Gulde & Partner

(56) References cited:
- EP-A1- 0 845 220
- WO-A2-2015/013327
- US-A1- 2013 192 623
- US-A1- 2014 069 424
- US-A1- 2015 144 147

## Description

### FIELD OF INVENTION

The present invention relates generally to electronic smoking devices and in particular electronic cigarettes.

### BACKGROUND OF THE INVENTION

An electronic smoking device, such as an electronic cigarette (e-cigarette), typically has a housing accommodating an electric power source (e.g. a single use or rechargeable battery, electrical plug, or other power source), and an electrically operable atomizer. The atomizer vaporizes or atomizes liquid supplied from a reservoir and provides vaporized or atomized liquid as an aerosol. Control electronics control the activation of the atomizer. In some electronic cigarettes, an airflow sensor is provided within the electronic smoking device, which detects a user puffing on the device (e.g., by sensing an under-pressure or an air flow pattern through the device). The airflow sensor indicates or signals the puff to the control electronics to power up the device and generate vapor. In other e-cigarettes, a switch is used to power up the e-cigarette to generate a puff of vapor.

The smoking experience depends on various aerosol attributes, such as throat hit, aerosol density, smoothness, which attributes in turn depend on the particle size in the aerosol.

US 2013/0192623 A1 discloses an electronic cigarette of the above-mentioned type, including at least one air inlet operable to deliver air to a central air passage upstream of a heater, and a mouth end insert having at least two diverging outlets. The electronic cigarette according to US 2013/0192623 A1 can also include an air flow diverter which directs incoming air away from a heating zone of the heater.

### SUMMARY OF THE INVENTION

In accordance with one aspect of the present invention there is provided an electronic smoking device according to claim 1, including a power supply, a liquid reservoir storing a liquid, and an atomizer. The atomizer is adapted to atomize the liquid stored in the liquid reservoir when operated by the power supply. An atomizing element is arranged in an atomizing chamber of the atomizer. The atomizing chamber includes an air inlet opening and an air outlet opening. The atomizing chamber is configured to be adaptable with respect to a distance between the air inlet opening and the atomizing element. The air inlet opening is provided in a base member that is sealingly arranged with respect to a tubular body that forms part of the atomizing chamber. The tubular body and the base member are configured so as to be movable relative to each other.

The characteristics, features and advantages of this invention and the manner in which they are obtained as described above, will become more apparent and be more clearly understood in connection with the following description of exemplary embodiments, which are explained with reference to the accompanying drawings.

### BRIEF DESCRIPTION OF THE DRAWINGS

In the drawings, same element numbers indicate same elements in each of the views:
- Figure 1A: is a schematic cross-sectional illustration of an exemplary e-cigarette;
- Figure 1B: is a schematic cross-sectional illustration of an exemplary e-cigarette of Fig. 1A with an adapted atomizing chamber;
- Figure 2: is a schematic cross-sectional illustration of an e-cigarette according to a second embodiment;
- Figure 3: is a schematic cross-sectional illustration of an e-cigarette according to a third embodiment;
- Figure 4: is a schematic cross-sectional illustration of an e-cigarette according to a fourth embodiment.

### DESCRIPTION OF THE PREFERRED EMBODIMENTS

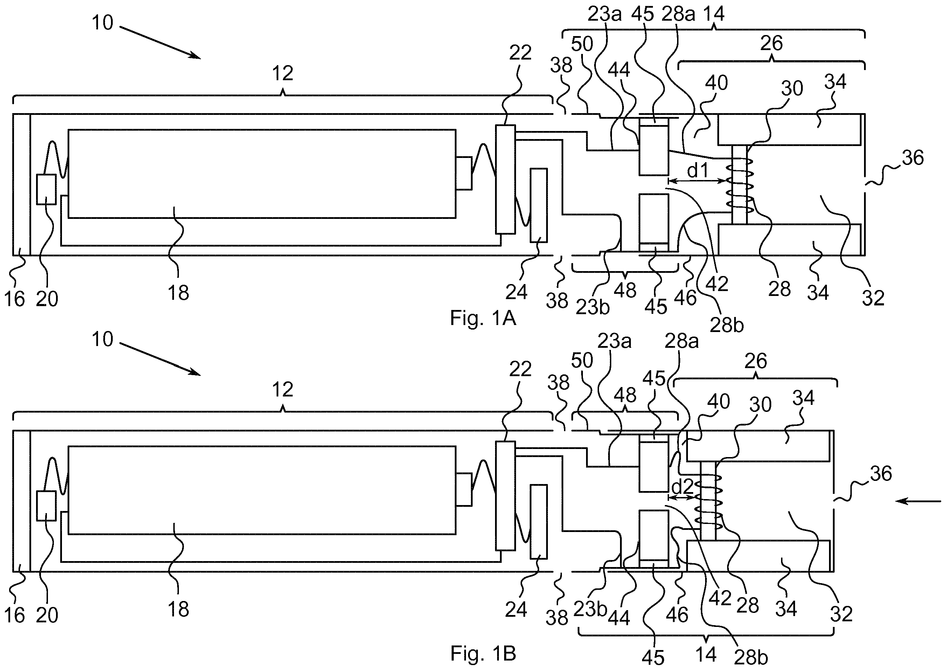

Throughout the following, an electronic smoking device will be exemplarily described with reference to an e-cigarette. As is shown in Figure 1A, an electronic smoking device 10 typically has a housing comprising a cylindrical hollow tube having an end cap 16. The cylindrical hollow tube may be a single-piece or a multiple-piece tube. In Figure 1A, the cylindrical hollow tube is shown as a multiple-piece structure having a power supply portion 12 and an atomizer/liquid reservoir portion 14. The atomizer/liquid reservoir portion 14 in turn comprises a base member 48 including first tubular body 50 and a base plate 44, and a second tubular body 46. The base member 48 and the second tubular body 46 are configured to be moveable relative to each other, as described below in detail with respect to Fig. 1B. Together the power supply portion 12 and the atomizer/liquid reservoir portion 14 form a cylindrical tube which can be approximately the same size and shape as a conventional cigarette, typically about 100 mm with a 7.5 mm diameter, although lengths may range from 70 to 150 or 180 mm, and diameters from 5 to 28 mm.

The power supply portion 12 and atomizer/liquid reservoir portion 14 are typically made of metal, e.g. steel or aluminum, or of hardwearing plastic and act together with the end cap 16 to provide a housing to contain the components of the e-cigarette 10. The power supply portion 12 and an atomizer/liquid reservoir portion 14 may be configured to fit together by a friction push fit, a snap fit, or a bayonet attachment, magnetic fit, or screw threads. The end cap 16 is provided at the front end of the power supply portion 12. The end cap 16 may be made from translucent plastic or other translucent material to allow an LED 20 positioned near the end cap to emit light through the end cap. The end cap can be made of metal or other materials that do not allow light to pass.

An air inlet may be provided in the end cap or anywhere along the length of the power supply portion, or at the connection of the power supply portion 12 and the atomizer/liquid reservoir portion 14. Figure 1A shows a pair of air inlets 38 provided at the intersection between the power supply portion 12 and the atomizer/liquid reservoir portion 14.

A battery 18, a light-emitting diode (LED) 20, control electronics 22 and optionally an airflow sensor 24 are provided within the cylindrical hollow tube power supply portion 12. The battery 18 is electrically connected to the control electronics 22, which are electrically connected to the LED 20 and the airflow sensor 24. In this example the LED 20 is at the front end of the power supply portion 12, adjacent to the end cap 16 and the control electronics 22 and airflow sensor 24 are provided in the central cavity at the other end of the battery 18 adjacent the atomizer/liquid reservoir portion 14.

The airflow sensor 24 acts as a puff detector, detecting a user puffing or sucking on the atomizer/liquid reservoir portion 14 of the e-cigarette 10. The airflow sensor 24 can be any suitable sensor for detecting changes in airflow or air pressure, such as a microphone switch including a deformable membrane which is caused to move by variations in air pressure. Alternatively the sensor may be a Hall element or an electro-mechanical sensor.

The control electronics 22 are also connected to an atomizing element 28 of the atomizer 26. In the example shown, the atomizer 26 comprises an atomizing chamber 40, in which an atomizing element in the form of heating coil 28 is arranged which is wrapped around a wick 30 extending across a central passage 32 of the atomizer/liquid reservoir portion 14. The wick 30 and heating coil 28 do not completely block the central passage 32. Rather an air gap is provided on either side of the heating coil 28 enabling air to flow past the heating coil 28 and the wick 30. The heating coil 28 may be transverse or parallel to the liquid reservoir 34.

The atomizing chamber 40 is defined by the second tubular body 46 that forms part of the atomizer/liquid reservoir portion 14 and a base member 44 arranged in the first tubular body 50 of the atomizer/liquid reservoir portion 14. The atomizing chamber 40 includes and air inlet opening 42 that is located in the base plate 44 of the base member 48 and an outlet opening 32, 36. As described below with reference to Fig. 1B, the distance d1 between the air inlet opening 42 of the atomizing chamber 40 and the atomizing element, i.e. the heating coil 28, is adaptable by adapting the atomizing chamber 40, in particular by moving the second tubular body 46 relative to the base member 48, into which the base plate 44 is fixedly arranged. The heating coil 28 is conductively connected to the control electronics 22 via flexible wires 28a, 28b and wires 23a and 23b, respectively. A conductive contact between wires 23a and 28a is provided via the base plate 44, which is electrically conductive. An insulating layer 45 insulates the base plate 44 and a conductive side wall of the tubular body 50, via which side wall the wires 23b and 28b are conductively connected. The wires 23 and 28 can e.g. be soldered to the respective elements 44, 50.

The atomizer may alternatively use other forms of heating elements, such as ceramic heaters, or fiber or mesh material heaters. Non-resistance atomizing elements such as sonic, piezo and jet spray may also be used in the atomizer in place of the heating coil.

The central passage 32 is surrounded by a cylindrical liquid reservoir 34 with the ends of the wick 30 abutting or extending into the liquid reservoir 34. The wick 30 may be a porous material such as a bundle of fiberglass fibers, with liquid in the liquid reservoir 34 drawn by capillary action from the ends of the wick 30 towards the central portion of the wick 30 encircled by the heating coil 28.

The liquid reservoir 34 may alternatively include wadding soaked in liquid which encircles the central passage 32 with the ends of the wick 30 abutting the wadding. In other embodiments the liquid reservoir 34 may comprise a toroidal cavity arranged to be filled with liquid and with the ends of the wick 30 extending into the toroidal cavity.

An air inhalation port 36 is provided at the back end of the atomizer/liquid reservoir portion 14 remote from the end cap 16. The inhalation port 36 may be formed from the cylindrical hollow tube atomizer/liquid reservoir portion 14 or maybe formed in an end cap.

In use, a user sucks on the e-cigarette 10. This causes air to be drawn into the electronic smoking device 10 via one or more air inlets, such as air inlets 38, and to be drawn through the air inlet opening 42 and the central passage 32 towards the air inhalation port 36. The change in air pressure which arises is detected by the airflow sensor 24, which generates an electrical signal that is passed to the control electronics 22. In response to the signal, the control electronics 22 activate the heating coil 28, which causes liquid present in the wick 30 to be vaporized creating an aerosol (which may comprise gaseous and liquid components) within the atomizing chamber 40. As the user continues to suck on the electronic smoking device 10, this aerosol is drawn through the outlet opening of the atomizing chamber 40, i.e. through the central passage 32 and inhaled by the user. At the same time the control electronics 22 also activate the LED 20 causing the LED 20 to light up which is visible via the translucent end cap 16 mimicking the appearance of a glowing ember at the end of a conventional cigarette. As liquid present in the wick 30 is converted into an aerosol more liquid is drawn into the wick 30 from the liquid reservoir 34 by capillary action and thus is available to be converted into an aerosol through subsequent activation of the heating coil 28.

Some e-cigarette are intended to be disposable and the electric power in the battery 18 is intended to be sufficient to vaporize the liquid contained within the liquid reservoir 34, after which the e-cigarette 10 is thrown away. In other embodiments the battery 18 is rechargeable and the liquid reservoir 34 is refillable. In the cases where the liquid reservoir 34 is a toroidal cavity, this may be achieved by refilling the liquid reservoir 34 via a refill port. In other embodiments the atomizer/liquid reservoir portion 14 of the e-cigarette 10 is detachable from the power supply portion 12 and a new atomizer/liquid reservoir portion 14 can be fitted with a new liquid reservoir 34 thereby replenishing the supply of liquid. In some cases, replacing the liquid reservoir 34 may involve replacement of the heating coil 28 and the wick 30 along with the replacement of the liquid reservoir 34. A replaceable unit comprising the atomizer 26 and the liquid reservoir 34 is called a cartomizer.

The new liquid reservoir 34 may be in the form of a cartridge having a central passage 32 through which a user inhales aerosol. In other embodiments, aerosol may flow around the exterior of the cartridge 32 to an air inhalation port 36.

Of course, in addition to the above description of the structure and function of a typical e-cigarette 10, variations also exist. For example, the LED 20 may be omitted. The airflow sensor 24 may be placed adjacent the end cap 16 rather than in the middle of the e-cigarette. The airflow sensor 24 may be replaced with a switch which enables a user to activate the e-cigarette manually rather than in response to the detection of a change in air flow or air pressure.

Different types of atomizers may be used. Thus for example, the atomizer may have a heating coil in a cavity in the interior of a porous body soaked in liquid. In this design aerosol is generated by evaporating the liquid within the porous body either by activation of the coil heating the porous body or alternatively by the heated air passing over or through the porous body. Alternatively the atomizer may use a piezoelectric atomizer to create an aerosol either in combination or in the absence of a heater.

Below, with respect to Fig. 1A, 1B, an adaptation of the atomizing chamber 40 with respect to the distance d1, d2 between the air inlet opening 42 and the atomizing element, i.e. the heating coil 28, is described. The air inlet opening 42 in the base plate 44 acts as a nozzle. The velocity of air entering the atomizing chamber 40 through the air inlet opening 42 during a puff is highest at an exit of the inlet opening 42, and decreases as a flow pathway expands into the atomizing chamber 40. The flow rate will be lowest at the widest part of the atomizing chamber 40 downstream the inlet opening 42. Thus, by adjusting the distance d1, d2 between the air inlet opening 42 and the heating coil 28, the velocity of the air passing the atomizing element 28 during a puff can be adjusted. This, in turn, influences the particle size in the aerosol, and consequently, different aerosol attributes, such as throat hit, smoothness, density, and flavor impression. Changing the velocity of the air passing over the coil will change the rate at which particles are removed from the aerosol generating area as they are being produced. This will adjust the concentration of particles in a given volume of air exiting the atomizer. In this way, the likelihood of particle growing through collision can be changed, which impacts the average size of the particles in the resulting aerosol.

As shown in Fig. 1A, 1B, according to a first embodiment, the base member 48 and second tubular body 46 are sealingly connected by means of a push fit fitting between the first tubular body 50 of the base member 48 and the second tubular body 46. Inside the first tubular body 50 the base plate 44 including the air inlet opening 42 of the atomizing chamber 40 is arranged. The base plate 44, fixedly arranged in the first tubular body 50, defines the atomizing chamber 40, together with the second tubular body 46. The base member 48 is partially arranged in the second tubular body 46, and the base member 48 and the second tubular body 46 are configured so as to be moved relative to each other, namely by pushing the second tubular body 46 in direction of the arrow in Fig. 1B (or by pulling the second tubular body 46 in the opposite direction).

By pushing the second tubular body 46, which can act as a mouthpiece of the electronic cigarette 10, in direction of the arrow (cf. Fig. 1B), the distance d1 between the heating coil 28 and the air inlet opening 42 (cf. Fig. 1A) is decreased to the new value d2 (cf. Fig. 1B). At the same time, the volume of the atomizing chamber 40 is also decreased. The distance d2 between the atomizing element 28 and the air inlet opening 42 can be increased again by simply pushing the mouthpiece in the opposite direction.

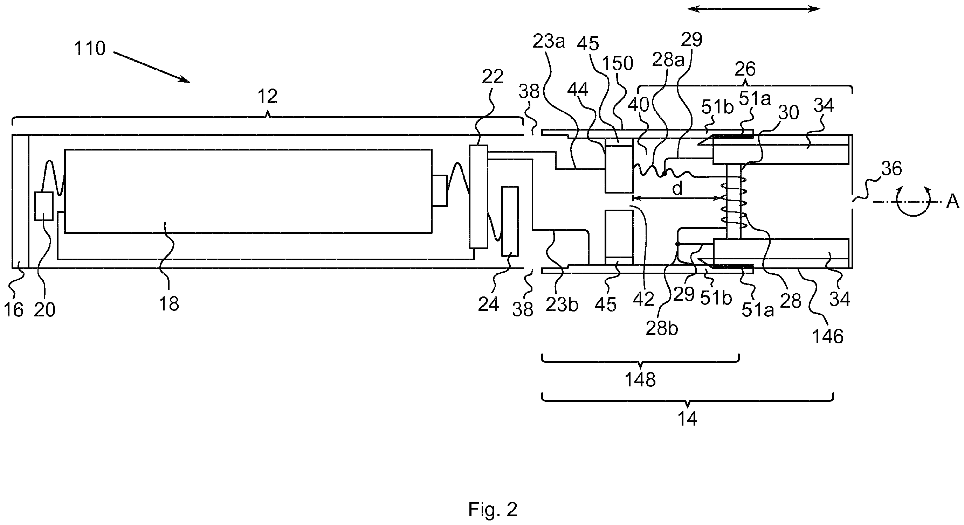

In Fig. 2, an electronic smoking device 110 according to a second embodiment is illustrated in a cross-sectional view. In contrast to the embodiment shown in Fig. 1A, 1B, the base member 148 is partially arranged on an outer surface of the second tubular body 146. Further, the connection between the base member 148 and the second tubular body 146 is a screw joint 51a, 51b, i.e. both an inner surface of the first tubular body 150 and an outer surface of the second tubular body 146 are threaded respectively. The distance d between the heating coil 28 and the air inlet opening 42 of the atomizing chamber 40, as well as the volume or length of the atomizing chamber 40, can be adapted by screwing in/out the second tubular body 146 into/out of the base member 148, i.e. by turning the second tubular body 146 around the axis A shown in Fig. 2. The conductive connection between the heating coil 28 and the control electronics 22 is basically the same as in Fig. 1A, 1B. However, in order to avoid twisting of the wires 28a, 28b when rotating the second tubular body 146 including the heating coil 28, the wires 28a, 28b are configured to only loosely contact the base plate 44 and the side wall of the tubular element 150, respectively. That is, the wires 28a, 28b are configured to slidably contact the base plate 44 and the side wall, respectively. In order to stabilize the arrangement of the wires 28a, 28b in the atomizing chamber 40, respective stabilizing elements 29 can be provided.

Needless to say that also the embodiment according to Fig. 1A, 1B can use a screw joint instead of a push fit fitting. On the other hand, a push fit fitting can be used in Fig. 2 instead of a screw joint.

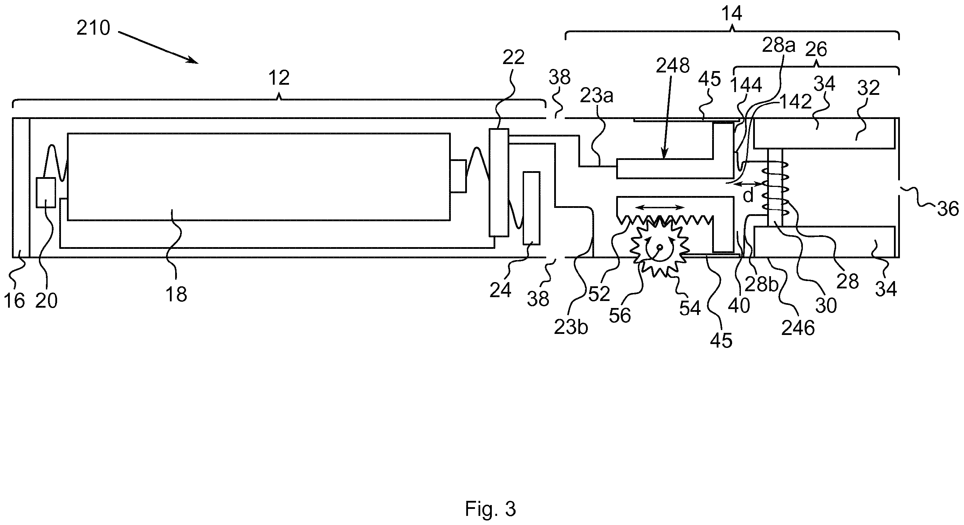

In Fig. 3 a third embodiment of an electronic smoking device 210 is illustrated in a cross-sectional view. In contrast to the embodiments of Fig. 1A, 1B and Fig. 2, the atomizer/liquid reservoir portion 14 is of a single-piece type and is formed by a tubular body 246. A base member 248 including a base plate 44 with an air inlet opening 142 in the form of a nozzle is sealingly and movably arranged inside the tubular body 246. The design of the nozzle 142 allows defining the air flow inside the atomizing chamber 40 during a puff. The connection between the base member 248 and the tubular body 246 is formed by means of a gear mechanism 52, 54. A rotatable dial in the form of a gear wheel 54 is rotatably - around an axis 56 - connected to the tubular body 246. The gear wheel 54 interlocks with a gear rack 52 that forms part of or is fixedly connected to the base member 248. By rotating the dial 54, the base member 248 can be moved along the longitudinal direction of the tubular member 246, as indicated by the arrow in Fig. 3. By moving the base member 248 in the tubular body 246, both the distance d between the atomizing element, i.e. the heating coil 28, and the air inlet opening 142 and the volume of the atomizing chamber 40 can be adapted by a user of the electronic smoking device 210.

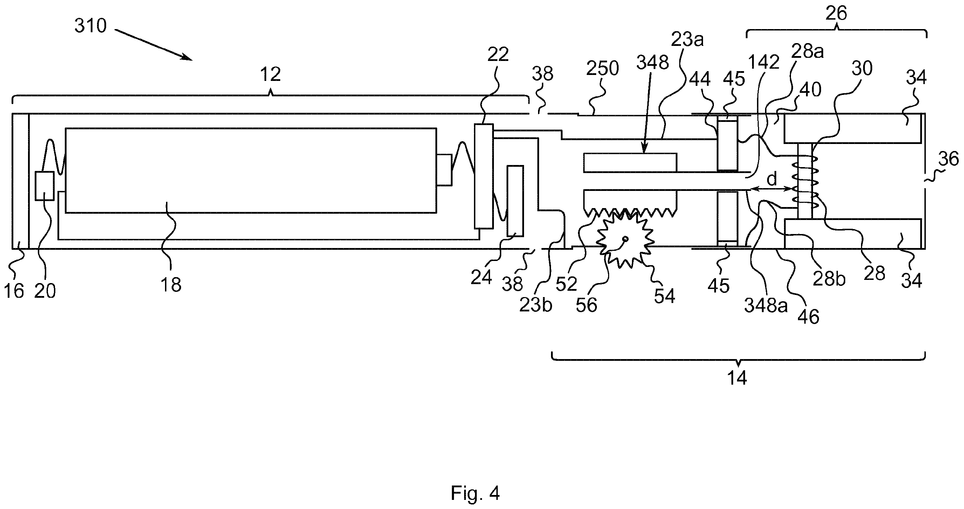

Fig. 4 illustrates a cross-sectional view of an electronic smoking device 310 according to a fourth embodiment. This embodiment is essentially a combination of the embodiment according to Fig. 1A, 1B and the embodiment according to Fig. 3. It will be apparent, that also the embodiment according to Fig. 2 can analogously be used in combination with the embodiment according to Fig. 3.

The embodiment according to Fig. 4 essentially serves to indicate that the distance d between the heating coil 28 and the air inlet opening 142 of the atomizing chamber 40 can be adjusted independent of the volume of the atomizing chamber 40, and vice versa. In contrast to the embodiment according to Fig. 3, the base member 348 does no longer comprise the base plate 44, which base plate 44 is fixedly attached to the first tubular body 250, as described with respect to Fig. 1A, 1B. The base member 348 comprises instead a tube-like extension 348a which is configured to be sealingly moved through the opening in the base plate 44. In other words, the air inlet opening 142 of the atomizing chamber 40 is now provided by the tube-like extension 348a, which can be moved with respect to the heating coil 28, essentially without changing the volume of the atomizing chamber 40, by rotating the gear wheel dial 54 that interlocks the base member 348 via the gear rack 52. On the other hand, the volume of the atomizing chamber 40 can be adjusted by pushing/pulling the second tubular body 46, as described with respect to Fig. 1A, 1B. Thereby, in general, also the distance d between the heating coil 28 and the air inlet opening 142 is changed. However, a respective alteration of the distance d can be corrected by respectively moving the base member 438 by the same amount by which the second tubular body 46 has been moved with respect to the first tubular body 250.

The above-described adaptations of the distance between the air inlet opening of the atomizing chamber and the atomizing element in the atomizing chamber, e.g. the heating coil, as well as the adjustment of the volume of the atomizing chamber can be deliberately controlled by a user of the respective electronic smoking device.

In summary, in one aspect the of the invention, an electronic smoking device according to claim 1 is disclosed. Said electronic smoking device has a power supply, a liquid reservoir storing a liquid, and an atomizer. The atomizer is adapted to atomize the liquid stored in the liquid reservoir when operated by the power supply. An atomizing element of the atomizer is arranged in an atomizing chamber including an air inlet opening and an air outlet opening. The atomizing chamber is configured to be adaptable with respect to a distance between the air inlet opening and the atomizing element.

According to an embodiment, the air inlet opening of the atomizing chamber is formed as a nozzle, in order to control the air flow in the atomizing chamber.

According to an embodiment, the atomizing chamber is further configured to be adaptable with respect to a volume of the atomizing chamber. According to specific embodiments, the volume of the atomizing chamber can be adjusted independent of an adjustment of the distance between the air inlet opening and the atomizing element.

According to an embodiment, the atomizing chamber is configured to be adaptable with respect to the distance between the air inlet opening and the atomizing element independent of an adaptation of a volume of the atomizing chamber.

According to the invention, the air inlet opening is provided in a base member that is sealingly arranged with respect to a tubular body that forms part of the atomizing chamber and contains the atomizing element. The tubular body and the base member are configured so as to be movable relative to each other, thereby changing the distance between the atomizing element and the inlet opening. The base member can at least partially be sealingly arranged in the tubular body. Alternatively, the base member can at least partially be sealingly arranged on an outer surface of the tubular body.

According to an embodiment, the distance between the air inlet opening and the atomizing element of the atomizer is adaptable my means of a screw joint between the base member and the tubular body.

According to an embodiment, the distance between the air inlet opening and the atomizing element of the atomizer is adaptable my means of a plug connection or push fit fitting between the base member and the tubular body.

According to an embodiment, the distance between the air inlet opening and the atomizing element of the atomizer is adaptable by means of a gear mechanism connecting the base member and the tubular body. The gear mechanism can include a rotatable dial fixedly connected to a gear-wheel that interlocks with a gear rack that is fixedly connected to the base member.

According to an embodiment, the atomizing element is formed as heating element or as non-resistance atomizer element. In particular, the atomizing element can be formed as heating coil that is wrapped around a wick that extends in the atomizing chamber.

According to a second aspect, an atomizer for an electronic smoking device according to claim 12 is provided. The atomizer is adapted to atomize liquid stored in a liquid reservoir of the electronic smoking device when operated by a power supply of the electronic smoking device. An atomizing element of the atomizer is arranged in an atomizing chamber including an air inlet opening and an air outlet opening. The atomizing chamber is configured to be adaptable with respect to a distance between the air inlet opening and the atomizing element. The air inlet opening is provided in a base member that is sealingly arranged with respect to a tubular body that forms part of the atomizing chamber and contains the atomizing element. The tubular body and the base member are configured so as to be movable relative to each other.

Preferred embodiments of a respective atomizer have already been described with respect to the electronic smoking device according to the first aspect.

According to a third aspect, an atomizer/liquid reservoir portion for an electronic smoking device is provided, which includes an atomizer according to the second aspect.

According to a fourth aspect, a cartomizer for an electronic smoking device is provide, which includes an atomizer according to the second aspect.

While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims.

### LIST OF REFERENCE SIGNS

- 10, 110, 210, 310: electronic smoking device
- 12: power supply portion
- 14: atomizer/liquid reservoir portion
- 16: end cap
- 18: battery
- 20: light-emitting diode (LED)
- 22: control electronics
- 23a, 23b: wire
- 24: airflow sensor
- 26: atomizer
- 28: heating coil
- 28a, 28b: flexible wire
- 29: stabilizing element
- 30: wick
- 32: central passage
- 34: liquid reservoir
- 36: air inhalation port
- 38: air inlets
- 40: atomizing chamber
- 42: air inlet opening
- 44, 144: base plate
- 45: insulating layer
- 46: (second) tubular body
- 48, 148, 248, 348: base member
- 50, 150, 250: first tubular body
- 51a, 51b: threaded surface
- 52: gear rack
- 54: gear wheel
- 56: axis
- 142, 242: air inlet opening in form of a nozzle
- 348a: tube-like extension
- d, d1, d2: distance between heating coil and air inlet opening

## Claims

1. An electronic smoking device (10; 110; 210; 310) comprising:
a power supply (18), a liquid reservoir (34) storing a liquid, and an atomizer (26) adapted to atomize the liquid stored in the liquid reservoir (34) when operated by the power supply (18), wherein an atomizing element (28) is arranged in an atomizing chamber (40) of the atomizer (26), the atomizing chamber (40) including an air inlet opening (42; 142; 242) and an air outlet opening (32, 36), wherein the atomizing chamber (40) is configured to be adaptable with respect to a distance (d; d1; d2) between the air inlet opening (42; 142; 242) and the atomizing element (28), **characterized in that** the air inlet opening (42) is provided in a base member (48; 148; 248; 348) that is sealingly arranged with respect to a tubular body (46; 146; 246) that forms part of the atomizing chamber (40), wherein the tubular body (46; 146; 246) and the base member (48; 148; 248; 348) are configured so as to be movable relative to each other.

2. The electronic smoking device (210; 310) according to claim 1, wherein the air inlet opening (142; 242) of the atomizing chamber (40) is formed as a nozzle (142; 242).

3. The electronic smoking device (10) according to claim 1 or 2, wherein the atomizing chamber (40) is configured to be adaptable with respect to a volume of the atomizing chamber (40).

4. The atomizing element (310) according to any one of the previous claims, wherein the atomizing chamber (40) is configured to be adaptable with respect to the distance (d) between the air inlet opening (242) and the atomizing element (28) independent of an adaptation of a volume of the atomizing chamber (40).

5. The electronic smoking (10; 110; 210; 310) device according to any one of the previous claims, wherein the base member (48; 248; 348) is at least partially sealingly arranged in the tubular body (46; 246) or wherein the base member (148) is at least partially sealingly arranged on an outer surface of the tubular body (146).

6. The electronic smoking device (110) according to any one of the previous claims, wherein the distance (d) between the air inlet opening (142) and the atomizing element (28) of the atomizer (26) is adaptable my means of a screw joint (51a, 51b) between the base member (148) and the tubular body (146).

7. The electronic smoking device (10) according to any one of the previous claims, wherein the distance (d1; d2) between the air inlet opening (42) and the atomizing element (28) of the atomizer (26) is adaptable my means of a plug connection or push fit fitting between the base member (48) and the tubular body (46).

8. The electronic smoking device (210; 310) according to any one of the previous claims, wherein the distance (d) between the air inlet opening (142; 242) and the atomizing element (28) of the atomizer (26) is adaptable my means of a gear mechanism (52, 54) connecting the base member (248; 348) and the tubular body (46; 246).

9. The electronic smoking device (210; 310) according to claim 8, wherein the gear mechanism includes a rotatable dial fixedly connected to a gear-wheel (54) that interlocks with a gear rack (52) that is fixedly connected to the base member (248; 348).

10. The electronic smoking device (10) according to any one of the previous claims, wherein the atomizing element (28) is formed as heating element or as non-resistance atomizer element.

11. The electronic smoking device (10) according to any one of claims 1 to 9, wherein the atomizing element is formed as heating coil (28) that is wrapped around a wick (30) that extends in the atomizing chamber (40).

12. An atomizer (26) for an electronic smoking device (10; 110; 210; 310), wherein the atomizer (26) is adapted to atomize liquid stored in a liquid reservoir (34) of the electronic smoking device (10; 110; 210; 310) when operated by a power supply (18) of the electronic smoking device (10; 110; 210; 310), wherein an atomizing element (28) is arranged in an atomizing chamber (40) of the atomizer (26), the atomizing chamber (40) including an air inlet opening (42; 142; 242) and an air outlet opening (32, 36), wherein the atomizing chamber (40) is configured to be adaptable with respect to a distance (d; d1; d2) between the air inlet opening (42; 142; 242) and the atomizing element (28), **characterized in that** the air inlet opening (42) is provided in a base member (48; 148; 248; 348) that is sealingly arranged with respect to a tubular body (46; 146; 246) that forms part of the atomizing chamber (40), wherein the tubular body (46; 146; 246) and the base member (48; 148; 248; 348) are configured so as to be movable relative to each other.

13. An atomizer/liquid reservoir portion (14) for an electronic smoking device (10; 110; 210; 310), including an atomizer (26) according to claim 12.

14. A cartomizer for an electronic smoking device (10; 110; 210; 310), including an atomizer (26) according to claim 12.

## Patentansprüche

1. Elektronische Rauchvorrichtung (10; 110; 210; 310), umfassend:
eine Energieversorgung (18), einen Flüssigkeitsbehälter (34), der eine Flüssigkeit speichert, und einen Zerstäuber (26), der dazu angepasst ist, die im Flüssigkeitsbehälter (34) gespeicherte Flüssigkeit zu zerstäuben, wenn er durch die Energieversorgung (18) betrieben wird, wobei in einer Zerstäubungskammer (40) des Zerstäubers (26) ein Zerstäubungselement (28) angeordnet ist, wobei die Zerstäubungskammer (40) eine Lufteinlassöffnung (42; 142; 242) und eine Luftauslassöffnung (32, 36) umfasst, wobei die Zerstäubungskammer (40) dazu gestaltet ist, in Bezug auf einen Abstand (d; d1; d2) zwischen der Lufteinlassöffnung (42; 142; 242) und dem Zerstäubungselement (28) anpassbar zu sein, **dadurch gekennzeichnet, dass** die Lufteinlassöffnung (42) in einem Basiselement (48; 148; 248; 348) bereitgestellt ist, das dichtend in Bezug auf einen rohrförmigen Körper (46; 146; 246) angeordnet ist, der einen Teil der Zerstäubungskammer (40) ausbildet, wobei der rohrförmige Körper (46; 146; 246) und das Basiselement (48; 148; 248; 348) dazu gestaltet sind, relativ zueinander bewegbar zu sein.

2. Elektronische Rauchvorrichtung (210; 310) nach Anspruch 1, wobei die Lufteinlassöffnung (142; 242) der Zerstäubungskammer (40) als Düse (142; 242) ausgebildet ist.

3. Elektronische Rauchvorrichtung (10) nach Anspruch 1 oder 2, wobei die Zerstäubungskammer (40) dazu gestaltet ist, in Bezug auf ein Volumen der Zerstäubungskammer (40) anpassbar zu sein.

4. Zerstäubungselement (310) nach einem der vorangehenden Ansprüche, wobei die Zerstäubungskammer (40) dazu gestaltet ist, in Bezug auf den Abstand (d) zwischen der Lufteinlassöffnung (242) und dem Zerstäubungselement (28) unabhängig von einer Anpassung eines Volumens der Zerstäubungskammer (40) anpassbar zu sein.

5. Elektronische Rauchvorrichtung (10; 110; 210; 310) nach einem der vorangehenden Ansprüche, wobei das Basiselement (48; 248; 348) zumindest teilweise dichtend im rohrförmigen Körper (46; 246) angeordnet ist oder wobei das Basiselement (148) zumindest teilweise dichtend an einer Außenfläche des rohrförmigen Körpers (146) angeordnet ist.

6. Elektronische Rauchvorrichtung (110) nach einem der vorangehenden Ansprüche, wobei der Abstand (d) zwischen der Lufteinlassöffnung (142) und dem Zerstäubungselement (28) des Zerstäubers (26) anhand einer Schraubverbindung (51a, 51b) zwischen dem Basiselement (148) und dem rohrförmigen Körper (146) anpassbar ist.

7. Elektronische Rauchvorrichtung (10) nach einem der vorangehenden Ansprüche, wobei der Abstand (d1; d2) zwischen der Lufteinlassöffnung (42) und dem Zerstäubungselement (28) des Zerstäubers (26) anhand einer Steckverbindung oder Schiebesitzverbindung zwischen dem Basiselement (48) und dem rohrförmigen Körper (46) anpassbar ist.

8. Elektronische Rauchvorrichtung (210; 310) nach einem der vorangehenden Ansprüche, wobei der Abstand (d) zwischen der Lufteinlassöffnung (142; 242) und dem Zerstäubungselement (28) des Zerstäubers (26) anhand eines Getriebemechanismus (52, 54) anpassbar ist, der das Basiselement (248; 348) und den rohrförmigen Körper (46; 246) verbindet.

9. Elektronische Rauchvorrichtung (210; 310) nach Anspruch 8, wobei der Getriebemechanismus eine drehbare Wählscheibe einschließt, die mit einem Zahnrad (54) fest verbunden ist, das mit einer mit dem Basiselement (248; 348) fest verbundenen Zahnstange (52) verzahnt ist.

10. Elektronische Rauchvorrichtung (10) nach einem der vorangehenden Ansprüche, wobei das Zerstäubungselement (28) als Heizelement oder als widerstandsfreies Zerstäuberelement ausgebildet ist.

11. Elektronische Rauchvorrichtung (10) nach einem der Ansprüche 1 bis 9, wobei das Zerstäubungselement als Heizspule (28) ausgebildet ist, die um einen sich in der Zerstäubungskammer (40) erstreckenden Docht (30) gewickelt ist.

12. Zerstäuber (26) für eine elektronische Rauchvorrichtung (10; 110; 210; 310), wobei der Zerstäuber (26) dazu angepasst ist, in einem Flüssigkeitsbehälter (34) der elektronischen Rauchvorrichtung (10; 110; 210; 310) gespeicherte Flüssigkeit zu zerstäuben, wenn er durch eine Energieversorgung (18) der elektronischen Rauchvorrichtung (10; 110; 210; 310) betrieben wird, wobei in einer Zerstäubungskammer (40) des Zerstäubers (26) ein Zerstäubungselement (28) angeordnet ist, wobei die Zerstäubungskammer (40) eine Lufteinlassöffnung (42; 142; 242) und eine Luftauslassöffnung (32, 36) umfasst, wobei die Zerstäubungskammer (40) dazu gestaltet ist, in Bezug auf einen Abstand (d; d1; d2) zwischen der Lufteinlassöffnung (42; 142; 242) und dem Zerstäubungselement (28) anpassbar zu sein, **dadurch gekennzeichnet, dass** die Lufteinlassöffnung (42) in einem Basiselement (48; 148; 248; 348) bereitgestellt ist, das dichtend in Bezug auf einen rohrförmigen Körper (46; 146; 246) angeordnet ist, der einen Teil der Zerstäubungskammer (40) ausbildet, wobei der rohrförmige Körper (46; 146; 246) und das Basiselement (48; 148; 248; 348) dazu gestaltet sind, relativ zueinander bewegbar zu sein.

13. Zerstäuber-/Flüssigkeitsbehälterabschnitt (14) für eine elektronische Rauchvorrichtung (10; 110; 210; 310), umfassend einen Zerstäuber (26) nach Anspruch 12.

14. Cartomizer für eine elektronische Rauchvorrichtung (10; 110; 210; 310), umfassend einen Zerstäuber (26) nach Anspruch 12.

## Revendications

1. Dispositif de cigarette électronique (10 ; 110 ; 210 ; 310) comprenant :
une alimentation électrique (18), un réservoir à liquide (34) stockant un liquide, et un nébuliseur (26) conçu pour atomiser le liquide stocké dans le réservoir à liquide (34) lorsque actionné par l'alimentation électrique (18), un élément d'atomisation (28) étant disposé dans une chambre de nébulisation (40) de le nébuliseur (26), la chambre de nébulisation (40) incluant une ouverture d'admission d'air (42 ; 142 ; 242) et une ouverture de sortie d'air (32, 36), la chambre de nébulisation (40) étant configurée pour être adaptable relativement à une distance (d ; d1 ; d2) entre l'ouverture d'admission d'air (42 ; 142 ; 242) et l'élément d'atomisation (28), **caractérisé en ce que** l'ouverture d'admission d'air (42) est fournie dans un membre de base (48 ; 148 ; 248 ; 348) qui est disposé de façon étanche relativement à un corps tubulaire (46 ; 146 ; 246) qui forme une partie de la chambre de nébulisation (40), le corps tubulaire (46 ; 146 ; 246) et le membre de base (48 ; 148 ; 248 ; 348) étant configurés de sorte à être déplaçables l'un par rapport à l'autre.

2. Dispositif de cigarette électronique (210 ; 310) selon la revendication 1, l'ouverture d'admission d'air (142 ; 242) de la chambre de nébulisation (40) étant formée comme une buse (142 ; 242).

3. Dispositif de cigarette électronique (10) selon la revendication 1 ou 2, la chambre de nébulisation (40) étant configurée pour être adaptable relativement à un volume de la chambre de nébulisation (40).

4. Élément d'atomisation (310) selon l'une quelconque des revendications précédentes, la chambre de nébulisation (40) étant configurée afin d'être adaptable relativement à une distance (d) entre l'ouverture d'admission d'air (242) et l'élément d'atomisation (28), indépendamment d'une adaptation d'un volume de la chambre de nébulisation (40).

5. Dispositif de cigarette électronique (10 ; 110 ; 210 ; 310) selon l'une quelconque des revendications précédentes, le membre de base (48 ; 248 ; 348) étant au moins partiellement disposé de façon étanche dans le corps tubulaire (46 ; 246) ou le membre de base (148) étant au moins partiellement disposé de façon étanche sur une surface externe du corps tubulaire (146).

6. Dispositif de cigarette électronique (110) selon l'une quelconque des revendications précédentes, la distance (d) entre l'ouverture d'admission d'air (142) et l'élément d'atomisation (28) de le nébuliseur (26) étant adaptable au moyen d'un assemblage à vis (51a, 51b) entre le membre de base (148) et le corps tubulaire (146).

7. Dispositif de cigarette électronique (10) selon l'une quelconque des revendications précédentes, la distance (d1 ; d2) entre l'ouverture d'admission d'air (42) et l'élément d'atomisation (28) de le nébuliseur (26) étant adaptable au moyen d'un raccordement à fiche ou d'un raccord par emboîtement entre le membre de base (48) et le corps tubulaire (46).

8. Dispositif de cigarette électronique (210 ; 310) selon l'une quelconque des revendications précédentes, la distance (d) entre l'ouverture d'admission d'air (142 ; 242) et l'élément d'atomisation (28) de le nébuliseur (26) étant adaptable au moyen d'un mécanisme d'engrenage (52, 54) reliant le membre de base (248 ; 348) et le corps tubulaire (46 ; 246).

9. Dispositif de cigarette électronique (210 ; 310) selon la revendication 8, le mécanisme d'engrenage incluant un cadran pivotant relié de façon fixe à une roue dentée (54) qui s'enclenche dans une crémaillère (52) qui est reliée de façon fixe au membre de base (248 ; 348).

10. Dispositif de cigarette électronique (10) selon l'une quelconque des revendications précédentes, l'élément d'atomisation (28) étant formé comme élément chauffant ou comme élément de nébuliseur sans résistance.

11. Dispositif de cigarette électronique (10) selon l'une quelconque des revendications 1 à 9, l'élément d'atomisation étant formé comme un serpentin de réchauffage (28) qui est entouré autour d'une mèche (30) qui s'étend dans la chambre de nébulisation (40).

12. Nébuliseur (26) pour un dispositif de cigarette électronique (10 ; 110 ; 210 ; 310), le nébuliseur (26) étant conçu pour atomiser du liquide stocké dans un réservoir à liquide (34) du dispositif de cigarette électronique (10 ; 110 ; 210 ; 310) lorsque actionné par une alimentation électrique (18) du dispositif de cigarette électronique (10 ; 110 ; 210 ; 310), un élément d'atomisation (28) étant disposé dans une chambre de nébulisation (40) de le nébuliseur (26), la chambre de nébulisation (40) incluant une ouverture d'admission d'air (42 ; 142 ; 242) et une ouverture de sortie d'air (32, 36), la chambre de nébulisation (40) étant configurée pour être adaptable relativement à une distance (d ; d1 ; d2) entre l'ouverture d'admission d'air (42 ; 142 ; 242) et l'élément d'atomisation (28), **caractérisé en ce que** l'ouverture d'admission d'air (42) est fournie dans un membre de base (48 ; 148 ; 248 ; 348) qui est disposé de façon étanche relativement à un corps tubulaire (46 ; 146 ; 246) qui forme une partie de la chambre de nébulisation (40), le corps tubulaire (46 ; 146 ; 246) et le membre de base (48 ; 148 ; 248 ; 348) étant configurés de sorte à être déplaçables l'un par rapport à l'autre.

13. Portion de réservoir à liquide/d'nébuliseur (14) pour un dispositif de cigarette électronique (10 ; 110 ; 210 ; 310) incluant un nébuliseur (26) selon la revendication 12,

14. Cartomiseur pour un dispositif de cigarette électronique (10 ; 110 ; 210 ; 310) incluant un nébuliseur (26) selon la revendication 12.
